(19) **Europäisches Patentamt**
**European Patent Office**
**Office européen des brevets**

(11) **EP 1 083 824 B1**

(12) **EUROPÄISCHE PATENTSCHRIFT**

(45) Veröffentlichungstag und Bekanntmachung des Hinweises auf die Patenterteilung:
**18.03.2009 Patentblatt 2009/12**

(51) Int Cl.:
***A61B 5/00*** *(2006.01)*

(21) Anmeldenummer: **99936403.7**

(22) Anmeldetag: **02.06.1999**

(86) Internationale Anmeldenummer:
**PCT/DE1999/001635**

(87) Internationale Veröffentlichungsnummer:
**WO 1999/062398 (09.12.1999 Gazette 1999/49)**

(54) **KALIBRIERUNGSFLUID FÜR EINEN SENSOR ZUR MESSUNG EINES BLUTWERTES, SOWIE HERSTELLUNG DES KALIBRIERUNGSFLUIDS**

CALIBRATING FLUID FOR CALIBRATING A SENSOR FOR MEASURING A BLOOD VALUE, UTILIZATION OF THE FLUID AND METHOD FOR PRODUCING THE SAME

FLUIDE D'ETALONNAGE DESTINE A L'ETALONNAGE D'UN CAPTEUR POUR LA MESURE D'UNE VALEUR SANGUINE, UTILISATION DE CE FLUIDE ET SON PROCEDE DE PREPARATION

(84) Benannte Vertragsstaaten:
**AT BE CH CY DE DK ES FI FR GB GR IE IT LI LU MC NL PT SE**

(30) Priorität: **04.06.1998 DE 19825014**

(43) Veröffentlichungstag der Anmeldung:
**21.03.2001 Patentblatt 2001/12**

(73) Patentinhaber: **SPHERE Medical Limited Cambridge Cambridgeshire CB2 5GG (GB)**

(72) Erfinder: **GUMBRECHT, Walter D-91074 Herzogenaurach (DE)**

(74) Vertreter: **Gillard, Richard Edward et al Elkington and Fife LLP Prospect House 8 Pembroke Road Sevenoaks Kent TN13 1XR (GB)**

(56) Entgegenhaltungen:
**EP-A- 0 362 032          EP-A- 0 571 066
EP-B- 0 657 030          FR-A- 2 436 991**

**Beschreibung**

Die Erfindung betrifft ein Kalibrierungsfluid für eine Kalibrierung eines Sensors zur Messung eines Blutwerts, eine Anwendung des Fluids und ein Verfahren zur Herstellung des Fluid.

Aus EP 0 657 030 B1 ist ein Kalibrierungsfluid für eine Kalibrierung eines Sensors zur Messung eines Blutwerts bekannt, das einen biokompatiblen Elektrolyten, der beispielsweise aus einer Ringer-, Ringer-Lactat- oder Ringer-Acetat-Infusionslösung besteht, und eine dieser Lösung zugegebene biokompatible Kohlendioxidquelle aufweist, die in der Lösung Bikarbonationen ($HCO_3^-$-Ionen) in bestimmter Konzentration und Kohlendioxid ($CO_2$) mit einem bestimmten Partialdruck $pCO_2$ erzeugt.

Die Zusammensetzungen von als Infusionslösungen verwendeten biokompatiblen Elektrolyten sind den Produktbeschreibungen der verschiedenen pharmazeutischen Unternehmen wie beispielsweise Pharmacia, Braun, Fresenius, Baxter u.v.a. zu entnehmen. Die Zusammensetzung von Blut oder Blutplasma hinsichtlich pH-Wert, Kohlendioxid-Partialdruck $pCO_2$ und Konzentrationen der im Blut enthaltenen Ionen ist z.B. beschrieben in Koryta, "Medical and Biological Applications of Electrochemical Devices", John Wiley & Sons, 1980, Seite 82.

Bei einem konkreten Beispiel des Kalibrierungsfluids nach der EP 0 657 030 B1 (siehe dort Seite 4, Zeilen 2 bis 7) besteht das Fluid aus 500 ml Ringer-Lactat-Lösung, der als Kohlendioxidquelle 10 ml von 8,4%-igem $NaHCO_3$ zugegeben sind. Die zugegebene Menge an $NaHCO_3$ entspricht etwa 20 mmol/l, die zusätzlich in der Ringer-Lactat-Lösung enthalten ist und den instabilen pH-Wert einer solchen Lösung wenigstens zeitweilig stabilisiert.

$HCO_3^-$-Ionen sind in herkömmlichen physiologischen Elektrolyten wie Ringer-Lösungen nicht enthalten, dafür aber in Blutplasma. Die normale physiologische Konzentration der $HCO_3^-$-Ionen in Blutplasma liegt bei 24 mmol/l und kann innerhalb eines diesen Konzentrationswert enthaltenden Konzentrationsbereich schwanken, der noch als normal physiologisch angesehen wird.

Die beim konkreten Beispiel des Kalibrierungsfluids nach der EP 0 657 030 B1 dem ursprünglichen, von $HCO_3^-$-Ionen freien Elektrolyten zugegebenen 20 mmol/l $NaHCO_3$ erzeugen im Elektrolyten $HCO_3^-$-Ionen in einer Konzentration, die noch im normalen physiologischen Bereich der Konzentration der $HCO_3^-$-Ionen in Blutplasma liegt bei 37°C liegt.

Die Konzentration der $HCO_3^-$- Ionen ist bei dem konkreten Beispiel des Kalibrierungsfluids nach der EP 0 657 030 B1 bei den 37°C so groß, daß dieses Fluid nach der bekannten Henderson-Hasselbalch-Gleichung (siehe beispielsweise Müller-Plathe, "Säure-Basen-Haushalt und Blutgase", Thieme Verlag, 1982, z.B. Seite 32) bei dieser Temperatur einen pH-Wert von 7,95 und einen Wert des $pCO_2$ von $1,2 \cdot 10^3$ Pa (= 9 mmHg) aufweist, welche Werte über beispielsweise 18 Stunden stabil bleiben.

Durch Zugabe eines anderen Mittels zur Regulierung des pH-Wertes, beispielsweise Natrium- oder Kaliumphosphat, kann der pH-Wert des Fluids auf etwa 7,1 abgesenkt werden, so daß es möglich ist, den $pCO_2$ auf über $7,33 \cdot 10^3$ Pa (= 55 mmHg) zu halten. In diesem Fall liegt aber kein Fluid nach dem Oberbegriff des Anspruchs 1 mehr vor, da die Konzentration der $HCO_3^-$-Ionen nicht im normalen physiologischen Bereich dieser Konzentration, beispielsweise im Bereich 24 mmol/l $\pm$ 5 mmol/l liegen.

In den pH-Bereich zwischen 7,1 und 7,95 fällt der normale physiologische pH-Wert von Blut, der in vielen medizinischen Lehrbüchern bei 37°C mit 7,41 angegeben ist.

Biokompatible bzw. physiologische Elektrolyten wie Ringer-Lösungen werden oft in Kunststoffbeuteln gelagert und sind luftgesättigt. Bei 22°C und $760 \cdot (4/3) \cdot 10^2$ Pa (760 mmHg) Luftdruck ergibt sich für diese Elektrolyten eine Partialdruck-Zusammensetzung von

$$155 \cdot (4/3) \cdot 10^2 \text{ Pa } (155 \text{ mmHg) } pO_2$$

$$585 \cdot (4/3) \cdot 10^2 \text{ Pa } (585 \text{ mmHg) } pN_2$$

$$20 \cdot (4/3) \cdot 10^2 \text{ Pa } (20 \text{ mmHg) } pH_2O,$$

d.h
insgesamt

$$760 \cdot (4/3) \cdot 10^2 \text{ Pa } (760 \text{ mmHg).}$$

Dabei sind $pO_2$ der Partialdruck des Sauerstoffs, $pN_2$ der Partialdruck des Stickstoffs und $pH_2O$ der Partialdruck des Wassers.

Durch die Zugabe der 20 mol/l $NaHCO_3$ zu einem solchen Elektrolyten addiert sich zur Summe dieser Partialdrücke $9 \cdot (4/3) \cdot 10^2$ Pa (9 mmHg) $pCO_2$ oder, bei Vorhandensein des anorganischen Phosphatpuffers, $55 \cdot (4/3) \cdot 10^2$ Pa (55 mmHg) $pCO_2$. Dadurch wird der jeweils vorliegende und auf dem Fluid lastende atmosphärische Druck überschritten und es kann zu einem Ausgasen des Fluids, d.h. zu einer Blasenbildung im Fluid kommen.

Physiologische Elektrolyten wie Ringer-Lösungen weisen eine Ionenstärke auf, die üblicherweise gleich der normalen physiologischen Ionenstärke des Blutes gewählt, die bei 155 mmol/l liegt und innerhalb eines diesen Ionenstärkewert enthaltenden Ionenstärkebereich schwanken kann, der noch als normal physiologisch angesehen wird.

Die Zugabe der 20 mmol/l $NaHCO_3$ zu einem solchen Elektrolyten erhöht die Ionenstärke des daraus resultierenden Kalibrierungsfluids um 20 mmol/l auf unphysiologische Werte, die beispielsweise zwischen 165 mmol/l und 185 mmol/l liegen.

Auch ist in physiologischen Elektrolyten wie Ringer-Lösungen die Konzentration von $Na^+$-Ionen üblicherweise gleich der normalen physiologischen Konzentration des Blutes gewählt, die bei 140mmol/l liegt. Die Zugabe der 20 mmol/l $NaHCO_3$ zum Elektrolyten erhöht die Konzentration der $Na^+$-Ionen im daraus resultierenden Kalibrierungsfluid von den normalen 140 mmol/l auf unphysiologische 160 mmol/l.

Überdies weisen physiologische Elektrolyte wie Ringer-Lösungen eine Osmolarität auf, die gleich der normalen physiologischen Osmolarität von Blutplasma gewählt ist, die bei 295 mosmol/l liegt und innerhalb eines diesen Osmolaritätswert enthaltenden Osmolaritätsbereich schwanken kann, der noch als normal physiologisch angesehen wird.

Die Zugabe der 20 mmol/l $NaHCO_3$ zu einem solchen Elektrolyten erhöht die Osmolarität des daraus resultierenden Kalibrierungsfluids um $2 \cdot 20$ mosmol/l = 40mosmol/l auf unphysiologische Werte, die beispielsweise zwischen 315 mosmol/l und 355 mosmol/l liegen.

Das bekannte Kalibrierungsfluid ist infundierbar und es können mit diesem Fluid Sensoren zur Messung des $pCO_2$ von Blut nicht nur in vitro sondern auch in vivo kalibriert werden.

EP 0362 032 A2 beschreibt eine Kalibrierungslösung die zur Messung von Ionenkonzentrationen mit einem pH Sensor oder anderen Ionensensoren mit Festkörper-Elektroden in Flüssigkeiten, wie zum Beispiel Blut angewendet wird. Die Ionenstärke der Kalibrierungslösung wird durch Zufügung von NaCl zu einer handelsüblichen Pufferlösung angepasst, so dass sie im Wesentlichen mit der Ionenstärke der Messlösung identisch ist.

FR 2 436 991 A beschreibt Kalibrierungslösungen mit drei $O_2$ und $CO_2$ Partialdrücke im Gleichgewicht mit drei verschiedenen pH-Werten. Die Partialdrücke der Gase korrespondieren zu den Werte von normalem, alkalischem und saurem Blut.

EP 0 571 066 beschreibt eine Kalibrierungsflüssigkeit für einen intravasculären Blutsensor. Die Kalibrierungsflüssigkeit ist eine wässerige Lösung die eine erste Pufferkomponente von 10-35 mM Bicarbonationen und eine zweite Pufferkomponente enthält, um den pH der Lösung im wesentlichen auf einen neutralen pH einzustellen.

EP 0 657 030 beschreibt biokompatibe Kalibrierurrgslösungen für die individuelle Kalibration von Sensoren in einer Gruppe von Sensoren für die gleichzeitigen Messung von Blutchemiewerten.

Aufgabe der Erfindung ist es, ein Kalibrierungsfluid bereitzustellen das eine Kalibrierung des Sensors mit einer im Vergleich zu einem bekannten Kalibrierungsfluid höheren Präzision ermöglicht.

Diese Aufgabe wird durch das im Anspruch 1 angegebene Kalibrierungsfluid gelöst.

In den im Anspruch 1 angegebenen Bereich von 24 mmol/l $\pm$ 5 mmol/l der Konzentration der Bikarbonat-Ionen fällt der normale physiologische Bereich der Konzentration der Bikarbonat-Ionen in Blutplasma und in den angegebenen Bereich von 155 mmol/l $\pm$ 10 mmol/l fällt der normale physiologischer Bereich der Ionenstärke des Blutes, und es kann zweckmäßig sein, für die Konzentration der Bikarbonat-Ionen einen möglichst nahe bei 24 mmol/l liegenden Wert und für die Ionenstärke einen möglichst nahe bei 155 mmol/l liegenden Wert zu wählen.

Das aus EP 0 657 030 B1 bekannte konkrete Beispiel eines Kalibrierungsfluids und das erfindungsgemäße Kalibrierungsfluid unterscheiden sich im wesentlichen dadurch voneinander, daß beim bekannten Kalibrierungsfluids die Ionenstärke nicht wie beim erfindungsgemäße Kalibrierungsfluid im Bereich von 155 mmol/l $\pm$ 10 mmol/l liegt, sondern außerhalb dieses Bereichs.

Das erfindungsgemäße Kalibrierungsfluid beruht auf der aus der Nernst'schen Gleichung (siehe Koryta, Seiten 13 ff) resultierenden Erkenntnis, daß bei potentiometrischer Bestimmung des unbekannten pH-Wertes und/oder einer unbekannten Konzentration einer Ionensorte einer Flüssigkeit wie beispielsweise Blut mit Hilfe einer Flüssigkeit eines bekannten pH-Wertes und/oder einer bekannten Konzentration dieser Ionensorte nur dann die Konzentrationen der beiden Flüssigkeiten unmittelbar miteinander verglichen werden können und ein richtiges Konzentrationsergebnis bezüglich der unbekannten Flüssigkeit erhalten werden kann, wenn die Ionenstärken der beiden Flüssigkeiten von vorneherein zumindest annähernd zueinander gleich sind.

Da beim erfindungsgemäßen die Ionenstärke im Bereich 155 mmol/l $\pm$ 10 mmol/l liegt, weist dieses Fluid im wesentlichen die gleiche Ionenstärke wie das Blut auf. Eine Abweichung der Ionenstärke des erfindungsgemäßen Kalibrierungsfluids vom tatsächlich vorliegenden Wert der normalen physiologischen Ionenstärke des Blutes ist in bezug auf eine ausreichende potentiometrische Meßgenauigkeit zulässig, solange die Ionenstärke des Fluids im Bereich 155 mmol/l $\pm$ 10 mmol/l bleibt.

Liegt dagegen die Ionenstärke des Kalibrierungsfluids außerhalb des Bereich 155 mmol/l $\pm$ 10 mmol/l, können Meßfehler auftreten, die das potentiometrische Meßergebnis verfälschen.

Bevorzugter und vorteilhafterweise ist das erfindungsgemäße Kalibrierungsfluid so ausgebildet, daß die im Blut enthaltenen Ionensorten im Fluid in so wenig von den normalen physiologischen Konzentrationen dieser Ionensorten im Blut abweichenden Konzentrationen vorhanden, daß bei einem Kontakt zwischen dem Fluid und dem Blut an der Grenzfläche zwischen dem Fluid und dem Blut eine Diffusionsspannung von weniger als 1 mV auftritt (Anspruch 2).

Diese Maßnahme beruht auf der aus der Hendersor-Gleichung (siehe Koryta, Seite 21f) resultierenden Erkenntnis, daß sich bei einer nicht vernachlässigbar großen Diffusionsspannung zwischen Fluid und Blut diese Spannung zumindest dort als Meßfehler auswirkt, wo das Kalibrierungsfluid als ein Brükkenelektrolyt zwischen einer Referenzelektrode und dem Blut verwendet wird, und daß dieser Meßfehler vernachlässigbar ist, wenn die Diffusionsspannung kleiner als 1 mV ist.

Eine nicht vernachlässigbar große Diffusionsspannung an der Grenze zwischen Fluid und Blut tritt beispielsweise auf, wenn die ionale Zusammensetzung des Fluids nicht in den Bereich der normalen physiologischen ionalen Zusammensetzung des Bluts fällt, wobei es bereits genügt, wenn die Konzentration nur einer einzigen Ionensorte des Fluids, beispielsweise die der $Na^+$-Ionen ausreichend weit außerhalb des Bereichs der normalen physiologischen Konzentration dieser Ionensorte im Blut liegt.

Bevorzugter und vorteilhafterweise weist das erfindungsgemäße Kalibrierungsfluid eine Osmolarität auf, die im Bereich 295 mosmol/l $\pm$ 20 mosmol/l liegt (Anspruch 3), in den der normale physiologische Bereich der Osmolarität von Blutplasma fällt. Dies hat den Vorteil, daß die Osmolarität des Fluids im Bereich einer Osmolarität eines Sensors zur Messung eines Blutwertes liegt und folglich beim Kontakt des Fluids mit diesem Sensor keine störenden Effekte durch osmotische Druckunterschiede auftreten. Es kann zweckmäßig sein, für die Osmolarität einen möglichst nahe bei 295 mosmol/l liegenden Wert zu wählen.

Das erfindungsgemäße Fluid kann vorteilhafterweise einen pH-Wert im Bereich zwischen 6,6 und 8,0 aufweisen (Anspruch 4), der vorteilhafterweise größer ist als der aus EP 0 657 030 B1 bekannte Bereich zwischen 7,1 und 7,95.

Eine besonders vorteilhafte und bevorzugte Ausführung eines erfindungsgemäßen Kalibrierungsfluids weist einen physiologischen organischen Puffer auf (Anspruch 5), der vorzugsweise durch ein bestimmtes Tris/TrisH$^+$-Verhältnis (Anspruch 6) und/oder ein bestimmtes R-PO$_4$$^{2-}$/R-HPO$_4$$^-$-Verhältnis definiert ist (Anspruch 7). R steht für einen wählbaren organischen Rest.

Das erfindungsgemäße Kalibrierungsfluid wird wie die üblicherweise verwendeten Kalibrierungsfluide meist bei Raumtemperatur (Standardwert 22°C) so hergestellt und gelagert, daß es bei dieser Temperatur eine Summe aller Partialdrücke aufweist, die gleich dem bei der Lagerung auf das Fluid lastenden Druck ist, der üblicherweise der lokale Atmosphärendruck (Standardwert $760 \cdot (4/3) \cdot 10^2$ Pa = 760 mmHg) ist.

In der Sensoreinrichtung, beispielsweise in einem Durchströmkanal dieser Einrichtung wird das Kalibrierungsfluid zweckmäßigerweise auf 37°C erwärmt, um eine Kalibrierung eines Sensors bei der Standard-Körpertemperatur sicherzustellen, bei der auch die Blutwerte gemessen werden.

Die Erwärmung des Kalibrierungsfluids von 22°C auf 37°C in der Sensoreinrichtung erfolgt schlagartig und ohne die Möglichkeit eines Gasaustausches mit der Umgebungsluft. Dies hat eine schlagartige Erhöhung der Summe aller Partialdrücke des Fluids zur Folge.

Ist die erhöhte Summe aller Partialdrücke des Fluids größer als der auf das Fluid lastende Druck, kann dies zu einem Ausgasen des Fluids und zu einer Bläschenbildung im Fluid führen. Dies ist eine Möglichkeit der Entstehung von Gasbläschen, die am Sensor selbst entstehen und/oder mit dem Fluid zum Sensor transportiert werden können.

Eine andere Möglichkeit der Entstehung von Gasbläschen am Sensor ist beispielsweise ein Einschluß von Luft oder eines anderen Gases im Kalibrieruhgsfluid und Transport dieses eingeschlossenen Gases zusammen mit dem Fluid zum Sensor.

In einem am Sensor festsitzenden Gasbläschen herrscht ein Bläschendruck, der gleich dem am Sensor auf dem Fluid lastenden Druck ist. Letzterer ist bei Sensoreinrichtungen, bei welchen die Blutwerte in vitro gemessen werden, üblicherweise gleich dem bei der Lagerung auf das Fluid lastenden Druck, meist der lokale Atmosphärendruck, bei Sensoreinrichtungen, bei welchen die Blutwerte in vivo gemessen werden, üblicherweise gleich dem bei der Lagerung auf das Fluid lastenden Druck zuzüglich des mittleren Blutdrucks (annähernd $100 \cdot (4/3) \cdot 10^2$ Pa = 100 mmHg) im Blutgefäß, mit dem der Sensor in Verbindung steht.

Gasblasen am Sensor während der Kalibrierung können zu erheblichen Kalibrierungsfehlern führen, die unkontrollierbare Meßfehler bei einer nachfolgenden Blutwertemessung nach sich ziehen.

Bei einem erfindungsgemäßen Kalibrierungsfluids kann vorteilhafterweise die Gefahr einer Entstehung von Kalibrierungsfehlern durch Gasbläschen dadurch herabgesetzt werden, daß die Summe aller Partialdrücke des Fluids kleiner als ein Druck ist, der auf das Fluid lastet (Anspruch 8).

Diese Maßnahme stellt vorteilhafterweise sicher, daß im Fluid keine Gasblasen durch Ausgasen des Fluids entstehen oder bereits vorhandene Gasblasen, beispielsweise eingeschlossene Luft, durch Absorption im Fluid verschwinden. Dies gilt insbesondere auch dann, wenn das Fluid zur Kalibrierung des Sensors bevorzugterweise am Sensor auf 37°C erwärmt wird, um eine Kalibrierung bei der Standard-Körpertemperatur zu gewährleisten.

Die Summe aller Partialdrücke des Fluids sollte zumindest kleiner als der Druck sein, der am Sensor auf das Fluid lastet. Dies gewährleistet, daß wenigstens am Sensor keine Gasbläschen entstehen können.

Sind in diesem Fall an anderer Stelle als am Sensor Gasbläschen im Fluid entstanden und gelangen diese zum Sensor, werden sie andererseits am Sensor vom Fluid absorbiert und verschwinden vorteilhafterweise von selbst, da ja am Sensor der auf das Fluid lastende Druck größer als die Summe aller Partialdrücke des Fluids ist.

An der anderen Stelle können Gasbläschen im Fluid durch Gaseinschluß oder, weil an dieser Stelle die Summe aller Partialdrücke des Fluids größer als der auf das Fluid lastende Druck ist, durch Ausgasen des Fluids entstehen.

Ein Ausgasen des Fluids läßt sich absolut verhindern, wenn die Summe aller Partialdrücke des Fluids kleiner als der kleinste auf das Fluid lastende Druck gewählt wird. In diesem Fall können im Fluid nirgendwo Gasblasen durch Ausgasen des Fluids entstehen und durch Gaseinschluß entstandene Gasbläschen werden nicht nur am Sensor, sondern überall sofort im Fluid absorbiert und verschwinden, da die Summe aller Partialdrücke des Fluids überall kleiner als der auf das Fluid lastende Druck ist.

Die Summe aller Partialdrücke des Fluids ist temperaturabhängig und steigt mit zunehmender Temperatur an. Diese Temperaturabhängigkeit ist zu berücksichtigen. Maßgebend ist die am Sensor herrschende Temperatur. Es muß gewährleistet sein, daß bei dieser Temperatur am Sensor die Summe aller Partialdrücke des Fluids kleiner als der Druck ist, der am Sensor auf das Fluid lastet.

Maßgebend ist die am Sensor herrschende Temperatur. Es muß gewährleistet sein, daß bei dieser Temperatur am Sensor die Summe aller Partialdrücke des Fluids kleiner als der Druck ist, der am Sensor auf dem Fluid lastet.

Die Summe aller Partialdrücke des erfindungsgemäßen Kalibrierungsfluids besteht zumindest aus dem Partialdruck eines Lösungsmittels, beispielsweise Wasser, und einem nicht verschwindenden Partialdruck $pCO_2$ des Kohlendioxids.

Vorteilhaft ist es, wenn im erfindungsgemäßen Kalibrierungsfluid der Partialdruck des $N_2$ bei 37°C kleiner als der normale physiologische Wert dieses Drucks im Blut bei dieser Temperatur ist (Anspruch 9). Der normale physiologische Wert des Partialrucks von $N_2$ im Blut bei 37°C beträgt etwa $573 \cdot (4/3) \cdot 10^2$ Pa (537 mmHg).

Diese Maßnahme bietet folgende Vorteile: Es ist günstig, die Summe aller Partialdrücke des Fluids möglichst klein im Vergleich zu dem auf das Fluid lastenden Druck zu wählen, da umso sicherer ein Ausgasen des Fluids verhindert und eine Absorption von Gasbläschen im Fluid bewirkt wird, je größer die Differenz zwischen dem größeren lastenden Druck und der kleineren Summe aller Partialdrücke des Fluids ist.

Der Partialdruck des neutralen Blutgases $N_2$ ist bei der Messung der Blutwerte einerseits nicht von Bedeutung, andererseits ist sein normaler physiologische Wert im Blut größer als die Summe aller übrigen normalen physiologischen Partialdrücke des Blutes, die für die Blutwertemessung von Bedeutung sind und aus diesem Grund auch im Fluid für eine Kalibrierung mit einem von null verschiedenen Partialdruckwert vorhanden sein sollten.

Es ist aus diesem Grund zweckmäßig, die Herabsetzung der Summe aller Partialdrücke des Fluids in erster Linie durch eine Erniedrigung des Partialdrucks von $N_2$ zu bewirken.

Besonders vorteilhaft ist es, auf $N_2$ im Fluid im wesentlichen ganz zu verzichten, so daß der $N_2$-Partialdruck des Fluids im wesentlichen gleich null ist (Anspruch 10). In diesem Fall wird vorteilhafterweise die größtmögliche Differenz zwischen dem am Sensor lastenden Druck und der Summe aller Partialdrücke des Fluids erreicht.

Ein erfindungsgemäßes Kalibrierungsfluid wird bevorzugter-und vorteilhafterweise in einer eine Referenzelektrode und eine Meßelektrode zur Messung eines Blutwertes aufweisenden potentiometrischen Meßanordnung als Brükken-Elektrolyt zwischen der Referenzelektrode und der Meßelektrode verwendet (Anspruch 11).

Das erfindungsgemäße Kalibrierungsfluid kann nicht wie das herkömmliche Kalibrierungsfluid aus einem herkömmlichen physiologischen Elektrolyten durch einfache Zugabe der Kohlendioxidquelle zu diesem Elektrolyten hergestellt werden Ein bevorzugtes und vorteilhaftes Verfahren zur einfachen Herstellung des erfindungsgemäße Kalibrierungsfluids ist im Anspruch 12 angegeben.

Das erfindungsgemäße Kalibrierungsfluid weist generell folgende Vorteile auf:

-    Das Fluid ermöglicht eine Kalibrierung eines Sensors mit einer im Vergleich zum bekannten Kalibrierungsfluid höheren Präzision sowohl in vivo als auch in vitro.

-    Da die Ionenstärke des Fluids im des Bereich 155 mmol/l ± 10 mmol/l gewählt ist, kann stets ein richtiges Konzentrationsergebnis bezüglich des Blutes erhalten werden.

-    Da die im Blut enthaltenen Ionensorten im Fluid in so wenig von den normalen physiologischen Konzentrationen dieser Ionensorten im Blut abweichenden Konzentrationen enthalten sind, daß bei einem Kontakt zwischen dem Fluid und dem Blut an der Grenzfläche zwischen dem Fluid und dem Blut eine Diffusionsspannung von weniger als 1 mV auftritt, ist diese Diffusionsspannung vernachlässigbar und es treten keine diffusionsspannungsbedingten Meßfehler auf, wenn das Kalibrierungsfluid als ein Brückenelektrolyt zwischen einer Referenzelektrode und dem Blut verwendet wird.

- Da im Fluid die Konzentration der Bikarbonat-Ionen im Bereich 24 mmol/l ± 5 mmol/l liegt, wird bewirkt, daß der Basenüberschuß (engl. Base excess) des Bluts durch das ins Blut infundierte Fluid nicht beeinträchtigt wird und folglich der im Blut tatsächlich vorhandene Basenüberschuß, gleichgültig ob er den bei 0 mmol/l liegenden normalen physiologischen Wert oder einen abnormen positiven oder negativen Wert aufweist, unverfälscht gemessen werden kann.

Der Basenüberschuß ist eine metabolische Kenngröße des Blutes, deren normale physiologische Wert gleich 0 mmol/l ist und die bei einer metabolischen Störung mehr oder weniger stark von 0 mmol/l abweichen kann. Ein von 0 mmol/l verschiedener Basenüberschuß des Blutes wird über die Niere nur sehr langsam, über Stunden normalisiert.

Durch die im Bereich 24 mmol/l ± 5 mmol/l liegende Konzentration an $HCO3^-$-Ionen im Fluid wird bei einer Infusion des Fluids vorteilhafterweise keine Störung des metabolischen Säure-Basen-Haushalts des Organismus hervorgerufen, die nur sehr langsam über die Nieren abgebaut werden kann.

- Der pH-Wert des Fluids kann stärker als bisher vom normalen physiologischen Wert 7,41 des Blutes abweichen. Dadurch kann vorteilhafterweise die Empfindlichkeit eines pH-Wert-Sensors mittels zweier verschiedener pH-Werte in einem größeren Meßbereich bestimmt werden.

Insbesondere kann der pH-Wert des Fluids im ganzen Bereich zwischen 6,6 und 8,0 vorteilhafterweise stark im Vergleich zum Bikarbonat- Puffer gepuffert sein, so daß er langzeitstabil eingestellt werden kann. Beispielsweise ist die Pufferwirkung von 20 mmol/l $NaHCO_3$ sehr gering, weil bei 37°C weniger als 0,3 mmol/l flüchtiges $CO_2$ im Fluid vorhanden sind. Die 20 mmol/l $NaHCO_3$ allein bewirken nur einen zeitweilig stabilen pH-Wert.

Das zur starken Pufferung des pH-Wertes des Fluids verwendete Puffersystem ist dann physiologisch unbedenklich, wenn das mit diesem Puffersystem versetzte Fluid die im Bereich 24 mmol/l ± 5 mmol/l liegende Konzentration an $HCO3^-$-Ionen aufweist. In diesem Fall wird das Blut mit dem infundierten stark gepufferten Fluid auch bei starker Abweichung des pH-Wertes des Fluids vom normalen pH-Wert 7,41 sehr schnell durch das respiratorische System, d.h. durch einmalige Passage der Lunge, auf den normalen physiologischen pH-Wert von 7,41 und den normalen physiologischen $pCO_2$ von $40 \cdot (4/3) \cdot 10^2$ Pa (40 mmHg) des Blutes gebracht. Der normale physiologischen $pCO_2$ ist eine respiratorische Kenngröße des Blutes.

Besonders geeignet zur starken Pufferung des die im Bereich 24 mmol/l ± 5 mmol/l liegende Konzentration an $HCO_3^-$-Ionen aufweisenden Fluids sind die physiologischen organischen Puffer, die den Vorteil haben, daß sie unbedenklich infundierbar sind.

- Das Fluid ist vorteilhafterweise für alle Patientengruppen physiologisch verträglich und ermöglicht eine hohe Präzision bei der Bestimmung der Blutwerte.

- Die Summe aller Partialdrücke des Fluids kann vorteilhafterweise kleiner als ein am Sensor auf das Fluid lastender Druck gewählt werden, so daß keine Gasbläschen im Fluid entstehen oder bereits vorhandene Gasbläschen im Fluid absorbiert werden, welche die Kalibrierung des Sensors stark verfälschen können.

- Mit dem Fluid können vorteilhafterweise die Sensoren zur Messung der folgenden Blutwerte kalibriert werden: $pCO_2$, pH-Wert und Konzentrationen aller im Blut oder Blutplasma enthaltenen Ionensorten wie $HCO_3^-$, $Na^+$, $K^+$, $Ca^{2+}$ $Mg^{2+}$, $Cl^-$, $SO_4^{2-}$, $CO_3^{2-}$ usw.

Die Erfindung wird in der nachfolgenden Beschreibung anhand der Figuren beispielhaft näher erläutert. Es zeigen:

Figur 1    in schematischer Darstellung eine Vorrichtung zur Messung von Blutwerten, deren Sensoren mit Hilfe eines erfindungsgemäßen Kalibrierungsfluids kalibriert werden können, und

Figur 2    in schematischer Darstellung einen Schnitt durch einen bekannten potentiometrischen Sensor mit einer Meß- und Referenzelektrode, bei dem ein erfindungsgemäßes Fluide als Brückenelektrolyt verwendet ist.

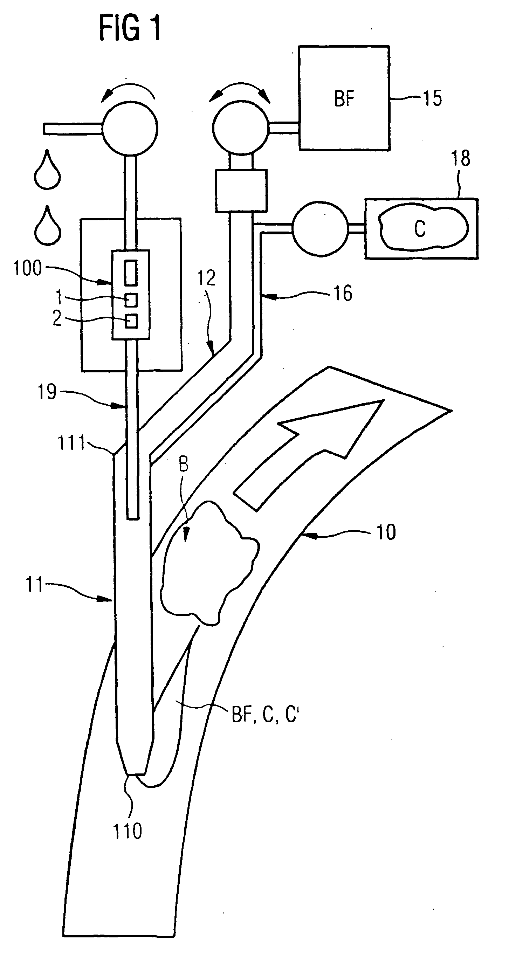

Im folgenden wird ein Anwendungsbeispiel des erfindungsgemäßen Kalibrierungsfluids beschrieben, das Bezug auf EP 0 790 499 A2 nimmt, aber nur in den relevanten Teilen wiedergegeben wird. Die in der vorliegenden Figur 1 dargestellte beispielhafte Vorrichtung basiert auf der Figur 1 und der dazugehörigen Beschreibung in EP 0 790 499 A2. Die in der vorliegenden Figur 1 verwendeten Bezugzeichen sind mit den entsprechenden Bezugzeichen der Figur 1 nach EP 0 790 499 A2 identisch und bezeichnen die gleichen Teile wie in EP 0 790 499 A2.

Es werden bei der vorliegenden Erfindung wie bei EP 0 790 499 A2 zur Eichung bzw. Kalibrierung eine Grundlinienflüssigkeit und zumindest eine Eichflüssigkeit verwendet. Gemäß der vorliegenden Figur 1 befindet sich die Grundlinienflüssigkeit BF in einem gasdichten Gefäß 15 in Form eines Kunststoffbeutels, die Eichflüssigkeit C in einem anderen gasdichten Gefäß 18 in Form eines Kunststoffbeutels. Beide Flüssigkeiten BF und C sind in den Gefäßen 15 und 18 bei Transport, Lagerung und im Einsatz vor Gasverlust geschützt.

Auf dem Weg vom jeweiligem Gefäß 15 bzw. 18 hin zu einer Sensoreinrichtung 100 muß jede Flüssigkeit je einen gasdurchlässigen Schlauch 12 bzw. 16 von 1,5 bis 2 m Länge durchfließen und ist dabei einem Gasverlust ausgesetzt.

Bedingt durch weitere Randbedingungen des Systems ist jedoch die Verweilzeit der Grundlinienflüssigkeit BF im Schlauch 12 größer als die der Eichflüssigkeit C im Schlauch 16. Dies hat zur Folge, daß der $pCO_2$ der Grundlinienflüssigkeit BF möglichst einen tief liegenden Wert haben muß, um den Gradienten zur praktisch $CO_2$-freien Luft möglichst klein zu halten.

In EP 0 790 499 A2 ist diesbezüglich von einem "ungenau bestimmten $pCO_2$-Wert kleiner 10 mmHg" der Grundlinienflüssigkeit BF die Rede.

Vorteilhaft ist demgegenüber ein möglichst genau bestimmter tief liegender Wert des $pCO_2$ der Grundlinienflüssigkeit BF.

Durch die Verwendung eines erfindungsgemäßen Kalibrierungsfluids mit einem durch ein Tris/TrisH$^+$-Verhältnis von 1:1 definierten organischen Puffer mit einem pK-Wert (siehe allgemeine Henderson-Hasselbalch-Gleichung) von 7,8 als Grundlinienflüssigkeit BF kann bei optimaler Pufferung vorteilhafterweise ein stabiler pH-Wert von 7,8 der Grundlinienflüssigkeit BF eingestellt werden, aus dem wegen Konzentration der $HCO_3^-$-Ionen im Bereich 24 mmol/l ± 5 mmol/l, die beim Fluid gegeben sind, gemäß der Henderson-Hasselbalch-Gleichung vorteilhafterweise ein genau bestimmter $pCO_2$-Wert von $14,1 \cdot (4/3) \cdot 10^2$ Pa (14,1 mmHg) der Grundlinienflüssigkeit BF eingestellt werden, was immer noch niedrig genug ist, um einen $CO_2$-Verlust an Luft zu minimieren. Außerdem wird die Stabilität des Wertes des $pCO_2$ noch durch die Gesamtkonzentration des Puffers beeinflußt. Gute Erfahrungen wurden mit einer Tris-Konzentration von 25 mmol/l gemacht.

Abgesehen von der Tatsache, daß bei Einhaltung der im Bereich 24 mmol/l ± 5 mmol/l liegenden Konzentration der $HCO_3^-$-Ionen bei Passage durch die Lunge sofort der normale pH-Wert 7,41 und normale $pCO_2$ von $40 \cdot (4/3) \cdot 10^2$ Pa (40 mmHg) erreicht werden, beträgt bei einer Tris-Konzentration von z.B. 25 mmol/l die Dosis von etwa 5 ml/h bis 3 mmol/Tag nur etwa 1 % der Dosis, die in Form von reinem alkalischen Tris bei der Theraphie einer metabolischen Azidose gegeben wird und einer Tris-Konzentration von 0,3 mol/l bei einem pH-Wert von= 10 und einem Basisüberschuß des Blutes von etwa 300 mmol/l entspricht.

Da die Eichflüssigkeit C eine kürzere Verweildauer im Schlauch 16 hat kann sie einen höheren Wert des $pCO_2$ aufweisen, der außerdem nicht die Stabilität wie bei der Grundlinienflüssigkeit BF aufweisen muß. Als Eichflüssigkeit C kann ein erfindungsgemäßes Kalibrierungsfluid verwendet werden, das im einfachsten Fall den normalen pH-Wert von 7,41 und gemäß der Henderson-Hasselbalch-Gleichung einen $pCO_2$ von $40 \cdot (3/4) \cdot 10^2$ Pa (40 mmHg), oder beispielsweise

| pH | $pCO_2 [\cdot(3/4) \cdot 10^2$ Pa oder mmHg] |
| --- | --- |
| 7,6 | 24 |
| 7,2 | 62 |
| 7,0 | 100 |

aufweisen kann.

Nachdem das $CO_2$-Pufferverhalten der Eichflüssigkeit C weniger kritisch ist als bei der Grundlinienflüssigkeit BF, muß ein eventuell eingesetzter pH-Puffer nicht unbedingt beim pK-Wert des Fluids festgesetzt werden. So wird hier vorzugsweise ein organischer physiologischer Puffer beispielsweise in Form eines Glycero-Phosphat-Puffers verwendet, der zwar einen pK-Wert von nur 6,2 aufweist, dafür aber erlaubt, Kalzium-Ionen einzusetzen, ohne wie bei anorganischen physiologischen Phosphat-Puffern Ausfällungen befürchten zu müssen.

Es seien nun drei konkrete Beispiele eines eines erfindungsgemäßen Kalibrierungsfluids und deren erfindungsgemäße Herstellung beschrieben:

Alle zur Herstellung der Beispiele verwendeten Substanzen entsprechen den im jeweiligen Anwendungsland gültigen Arzneimittelverordnungen.

Zur Herstellung des ersten konkreten Beispiels werden für beispielsweise einen Ansatz von 100 Litern Fluid als Ausgangsstoffe

557,99 g NaCl
37,28 g KCl
22,18 g $MgSO_4$ * $7H_2O$
133,63 g Na-Acetat * $3H_2O$
302,85 g Tris
207,50 g $NaHCO_3$
1292,35 g HCl (1 mol/l HCl)
97,946 kg Wasser

verwendet.

Die Salze und HCl-Lösung werden in Wasser gelöst und anschließend mit $CO_2$, $O_2$ abhängig von der Temperatur und Druck mit einem geeigneten Verhältnis dieser Gase so begast, daß die äquilibrierte Lösung bei 37°C einen $pCO_2$-Wert von 14.1 mmHg als auch einen geeigneten $pO_2$-Wert aufweist.

Aufgrund der pK-Werte 7.8 für Tris/TrisH⁺, 6.08 für $HCO_3^-$/$CO_2$ und dem molaren Löslichkeitskoeffizienten von $CO_2$ in Wasser von 0.0325 ergibt sich folgende Zusammensetzung des Fluids:

130,00 mmol/l $Na^+$
5,00 mmol/l $K^+$
0,90 mmol/l $Mg^{2+}$
12,50 mmol/l Tris
12,50 mmol/l $TrisH^+$
113,20 mmol/l $Cl^-$
0,90 mmol/l $SO_4^{2-}$
9,82 mmol/l $Acetat^-$
0,24 mmol/l $CO_3^{2-}$
24,00 mmol/l $HCO_3^-$
pH-Wert = 7,80

$$pCO_2 = 14,1 \ mmHg = 14,1 \cdot (4/3) \cdot 10^2 \ Pa$$

Dieses Fluid ist ein Ca-freies, Tris-gepuffertes Kalibrierfluid. Es enthält kein $N_2$, gast bei dem üblicherweise am Sensor auf dem Fluid lastenden Druck vorteilhafterweise nicht in Gasbläschen aus und/oder läßt anderweitig, beispielsweise durch Lufteinschluß entstandene Gasbläschen durch Absorption im Fluid verschwinden.

Das Fluid könnte auch $N_2$ enthalten das wie $CO_2$ und $O_2$ durch Begasen der Lösung eingebracht werden kann. Es empfiehlt sich auch in diesem Fall, die Summe aller Partialdrücke des Fluids und darüber hinaus den von null verschiedenen Partialdruck $pN_2$ des Stickstoffs so klein im Vergleich zu einem auf dem Fluid lastenden Druck zu wählen, daß kein Ausgasen des Fluids auftreten kann und/oder anderweitig entstandene Gasbläschen im Fluid absorbiert werden.

Mit diesem Fluid können die Sensoren für $pCO_2$, den pH-Wert und die Konzentration der im Blut vorhandenen Ionen $HCO_3^-$, $Na^+$, $K^+$, $Mg^{2+}$,$Cl^-$ $SO_4^{2-}$ und $CO_3^{2-}$ kalibriert werden. Bei dem in EP 0 790 499 A2 beschriebenen Kalibrierungsverfahren, bei dem vorteilhafterweise auch die Empfindlichkeit der Sensoren, insbesondere des $CO_2$-Sensors kalibriert werden kann, ist dieses beispielhafte Fluid besonders als Grundlinienflüssigkeit BF geeignet, da der $pCO_2$ nur $14,1 \cdot (4/3) \cdot 10^2$ Pa (14,1 mmHg) beträgt und ausreichend nahe beim Wert des $pCO_2$ der Luft liegt. Als Eichflüssigkeit C sollte ein anderes Fluid verwendet werden, das sich für den $CO_2$-Sensor im Wert des $pCO_2$, für den pH-Sensor im pH-Wert und/oder für einen Ionensorten-Sensor in der Konzentration dieser Ionensorte von der Grundlinienflüssigkeit BF unterscheidet, so daß der $CO_2$-Sensor, der pH-Sensor und/oder der Ionensorten-Sensor mit dieser Eichflüssigkeit C bezüglich der Grundlinienflüssigkeit BF kalibriert werden kann.

Zur Herstellung des zweiten konkreten Beispiels werden für beispielsweise einen Ansatz von 100 Litern Fluid als Ausgangsstoffe

556.93 g NaCl
37.28 g KCl
1.47 g $CaCl_2$ * $2H_2O$
22.18 g $MgSO_4$ * $7H_2O$
133.63 g Na-Acetat * $3H_2O$

302.85 g Tris
207.50 g NaHCO$_3$
1292.35 g (1 mol/l HCl)
97.946 kg Wasser

verwenden.

Die Salze und HCl-Lösung werden in Wasser gelöst und anschließend mit CO$_2$, O$_2$ abhängig von der Temperatur und Druck mit einem geeigneten Verhältnis dieser Gase so begast, daß die äquilibrierte Lösung bei 37°C einen pCO$_2$-Wert von 14.1 mmHg als auch einen geeigneten pO$_2$-Wert aufweist.

Aufgrund der pK-Werte 7.8 für Tris/TrisH$^+$, 6.08 für HCO$_3^-$/CO$_2$ und dem molaren Löslichkeitskoeffizienten von CO$_2$ in Wasser von 0.0325 ergibt sich folgende Zusammensetzung des Fluids:

129.80 mmol/l Na$^+$
5.00 mmol/l K$^+$
0.10 mmol/l Ca$^{2+}$
0.90 mmol/l Mg$^{2+}$
12.50 mmol/l Tris
12.50 mmol/l TrisH$^+$
113.20 mmol/l Cl$^-$
0.90 mmol/l SO$_4^{2-}$
9.82 mmol/l Acetat$^-$
0.24 mmol/l CO$_3^{2-}$
24.00 mmol/l HCO$_3^-$
pH-Wert = 7.80

$$pCO_2 = 14.1 \text{ mmHg} = 14{,}1 \cdot (4/3) \cdot 10^2 \text{ Pa}$$

Dieses Fluid ist ein Ca-haltiges, Tris-gepuffertes Kalibrierfluid. Es enthält kein N$_2$, gast bei dem üblicherweise am Sensor auf dem Fluid lastenden Druck vorteilhafterweise nicht in Gasbläschen aus und/oder läßt anderweitig, beispielsweise durch Lufteinschluß entstandene Gasbläschen durch Absorption im Fluid verschwinden.

Dieses Fluid könnte ebenfalls N$_2$ enthalten das wie CO$_2$ und O$_2$ durch Begasen der Lösung eingebracht werden kann. Es empfiehlt sich auch in diesem Fall, die Summe aller Partialdrükke des Fluids und darüber hinaus den von null verschiedenen Partialdruck pN$_2$ des Stickstoffs so klein im Vergleich zu einem auf dem Fluid lastenden Druck zu wählen, daß kein Ausgasen des Fluids auftreten kann und/oder anderweitig entstandene Gasbläschen im Fluid absorbiert werden.

Mit diesem Fluid können die Sensoren für pCO$_2$, den pH-Wert und die Konzentration der im Blut vorhandenen Ionen HCO$_3^-$, Na$^+$, K$^+$, Mg$^{2+}$ Ca$^{2+}$, Cl$^-$, SO$_4^{2-}$ und CO$_3^{2-}$ kalibriert werden. Bei dem in EP 0 790 499 A2 beschriebenen Kalibrierungsverfahren ist auch dieses beispielhafte Fluid besonders als Grundlinienflüssigkeit BF geeignet, da der pCO$_2$ nur 14,1·(4/3)·10$^2$ Pa (14,1 mmHg) beträgt und ausreichend nahe beim Wert des pCO$_2$ der Luft liegt. Als Eichflüssigkeit C sollte auch hier ein anderes Fluid verwendet werden, das sich für den CO$_2$-Sensor im Wert des pCO$_2$, für den pH-Sensor im pH-Wert und/oder für einen Ionensorten-Sensor in der Konzentration dieser Ionensorte von der Grundlinienflüssigkeit BF unterscheidet, so daß der CO$_2$-Sensor, der pH-Sensor und/oder der Ionensorten-Sensor mit dieser Eichflüssigkeit C bezüglich der Grundlinienflüssigkeit BF kalibriert werden kann.

Zur Herstellung des dritten konkreten Beispiels werden für beispielsweise einen Ansatz von 100 Litern Fluid als Ausgangsstoffe

533,85 g NaCl
29,82 g KCl
17,64 g CaCl$_2$ * 2H$_2$O
14,79 g MgSO$_4$ * 7H$_2$O
13,61 g Na-Acetat * 3H$_2$O
306,12 g Na$_2$-Glycero-Phosphat * 5H$_2$O
232,29 g NaHCO$_3$
532,38 g (1 mol/l HCl)
98,986 kg Wasser

verwendet.

Die Salze und HCl-Lösung werden im Wasser gelöst und anschließend mit $CO_2$, $O_2$ abhängig von der Temperatur und Druck mit einem geeigneten Verhältnis dieser Gase so begast, daß die äquilibrierte Lösung bei 37°C einen $pCO_2$-Wert von 112 mmHg als auch einen geeigneten $pO_2$-Wert aufweist.

Aufgrund der pK-Werte 6.2 für $R-PO_4^{2-}$/$R-HPO_4^-$, 6.08 für $HCO_3^-$/$CO_2$ und dem molaren Löslichkeitskoeffizienten von $CO_2$ in Wasser von 0.0325 ergibt sich folgende Zusammensetzung der Lösung:

140,00 mmol/l $Na^+$
4,00 mmol/l $K^+$
1,20 mmol/l $Ca^{2+}$
0,60 mmol/l $Mg^{2+}$
8,35 mmol/l $R-PO_4^{2-}$
1,65 mmol/l $R-HPO_4^-$
102,99 mmol/l $Cl^-$
0,60 mmol/l $SO_4^{2-}$
1,00 mmol/l $Acetat^-$
0,03 mmol/l $CO_3^{2-}$
24,00 mmol/l $HCO_3^-$
pH-Wert = 6,9

$$pCO_2 = 112 \text{ mmHg} = 14,1 \cdot (4/3) \cdot 10^2 \text{ Pa}$$

Dieses Fluid ist ein Ca-haltiges, Glycero-Phosphatgepuffertes_Kalibrierfluid. Es enthält kein $N_2$, gast bei dem üblicherweise am Sensor auf dem Fluid lastenden Druck vorteilhafterweise nicht in Gasbläschen aus und/oder läßt anderweitig, beispielsweise durch Lufteinschluß entstandene Gasbläschen durch Absorption im Fluid verschwinden.

Auch dieses Fluid könnte $N_2$ enthalten das wie $CO_2$ und $O_2$ durch Begasen der Lösung eingebracht werden kann. Es empfiehlt sich auch in diesem Fall, die Summe aller Partialdrükke des Fluids und darüber hinaus den von null verschiedenen Partialdruck $pN_2$ des Stickstoffs so klein im Vergleich zu einem auf dem Fluid lastenden Druck zu wählen, daß kein Ausgasen des Fluids auftreten kann und/oder anderweitig entstandene Gasbläschen im Fluid absorbiert werden

Mit diesem Fluid können die Sensoren für pCO2, den pH-Wert und die Konzentration der im Blut vorhandenen Ionen $HCO_3^-$, $Na^+$, $K^+$, $Mg^{2+}$ $Ca^{2+}$, $Cl^-$, $SO_4^{2-}$ und $CO_3^{2-}$ kalibriert werden. Bei dem in EP 0 790 499 A2 beschriebenen Kalibrierungsverfahren, bei dem auch die Empfindlichkeit der Sensoren, insbesondere des CO2-Sensors kalibriert werden kann, ist dieses beispielhafte Fluid besonders als Eichflüssigkeit C geeignet, da der $pCO_2$ mit $112 \cdot (4/3) \cdot 10^2$ Pa (112 mmHg) weit entfernt vom Wert des $pCO_2$ der Luft liegt. Als Grundlinienflüssigkeit BF kann in diesem Fall das erste oder zweite konkrete Beispiel des erfindungsgemäßen Fluids verwendet werden, das sich für den $CO_2$-Sensor im $pCO_2$, den pH-Sensor im pH-Wert und/oder für einen Ionensorten-Sensor in der Konzentration dieser Ionensorte von dieser Eichfüssigkeit C unterscheidet, so daß der $CO_2$-Sensor, der pH-Sensor und/oder der Ionensorten-Sensor mit dieser Eichflüssigkeit C jeweils bezüglich dieser Grundlinienflüssigkeit BF kalibriert werden kann..

Ein erfindungsgemäßes Kalibrierungsfluid kann wie in EP 0 790 499 A2 beschrieben durch eine in eine in ein Blutgefäß 10, beispielsweise eine Arterie, eingebrachte Kanüle 11 mit einer innerhalb des Blutgefäßes 10 im darin strömenden Blut B befindlichen offenen Spitze L10 und einem außerhalb des Blutgefäßes 10 befindlichen offenen Ende 111 und durch einen vom Innern der Kanüle 11 zur Sensoreinrichtung 100 führenden Flüssigkeitskanal 19 als Grundlinienflüssigkeit BF vom Schlauch 12 oder als Eichflüssigkeit C oder andere Eichflüssigkeit vom Schlauch 16 als zu jedem in der Sensoreinrichtung 100 enthaltenen Sensor 1, 2, usw. gebracht werden.

Die Kalibrierung eines Sensors 1, 2 usw. und eine Blutwertmessung mit diesem Sensor kann wie in EP 0 790 499 A2 beschrieben durchgeführt werden, d.h. es erfolgt eine Kalibrierung des Sensors 1, 2 usw. mit der Grundlinienflüssigkeic BF, mit der Eichflüssigkeit C und ggf. mit einer anderen Eichflüssigkeit, die sich für den $CO_2$-Sensor im Wert des pCO2, für den pH-Sensor im pH-Wert und/oder für einen Ionensorten-Sensor in der Konzentration dieser Ionensorte von der Eichflüssigkeit C unterscheidet und zur Kalibrierung einer Empfindlichkeitdieses Sensors dient. Ein Beispiel ist die nach EP 0 790 499 A2 aus der Eichflüssigkeit C gewonnene andere Eichflüssigkeit C', die sich von der Eichflüssigkeit C im Wert des $pCO_2$ unterscheidet und zur Kalibrierung der Empfindlichkeit des dortigen $CO_2$-Sensors 1 dient.

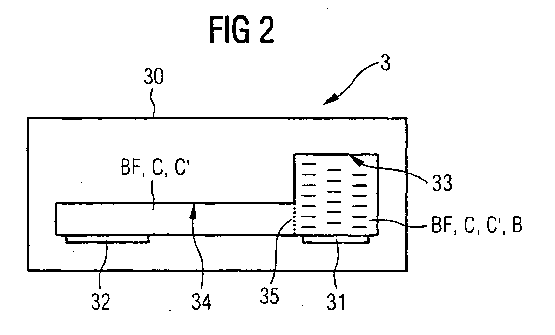

Der in der Figur 2 schematisch im Schnitt gezeigte potentiometrische Sensor 3, der in der Sensoreinrichtung 100 nach Figur 1 enthalten sein kann, besteht beispielsweise aus einem Gehäuse 30 mit einem Hohlraum, einer Meßelektrode 31 und einer Referenzelektrode 32.

Der Hohlraum besteht aus einer mit dem Flüssigkeitskanal 19 nach Figur 1 verbundenen Kammer 33 und einem an die Kammer 33 angeschlossenen und von dieser fortführenden Stichkanal 34.

Die Kammer 33 wird durch den Flüssigkeitskanal 19 zum Kalibrieren des Sensors 3 mit erfindungsgemäßem Kalibrierungsfluid und zum Messen eines Blutwertes mit Blut B gefüllt. Dieses Kalibrierungsfluid bildet die Basisflüssigkeit BF, Eichflüssigkeit C oder auch die andere Eichflüssigkeit, beispielsweise die Flüssigkeit C'.

Im Bereich der Kammer 33 ist die Meßelektrode 31 so angeordnet, daß sie mit der jeweiligen Füllung BF, C, C' oder B der Kammer 33 in Kontakt steht.

Die Referenzelektrode 32 ist in einem Abstand von der Kammer 33 und der Meßelektrode 31 im Bereich des Stichkanals so angeordnet, daß sie mit der Füllung des Stichkanals 34 in Kontakt steht. Diese Füllung besteht stets aus erfindungsgemäßem Kalibrierungsfluid, d.h. aus Basisflüssigkeit BF, Eichflüssigkeit C oder auch anderer Eichflüssigkeit, beispielsweise der Flüssigkeit C', da einer Messung eines Blutwertes stets eine Kalibrierung mit erfindungsgemäßer Kalibrierungsflüssigkeit vorausgeht, bei welcher der ganze Hohlraum 33 und 34 mit diesem Fluid gefüllt wird, bei der Messung des Blutwerte dagegen nur die Kammer 33 mit Blut B gefüllt wird, im Stichkanal 34 aber erfindungsgemäßes Kalibrierungsfluid verbleibt.

Dieses verbleibende Kalibrierungsfluid bildet einen Brücken-Elektrolyten, der die Referenzelektrode 32 mit dem Blut B und damit mit der Meßelektrode 31 verbindet. An der Grenzfläche 35 zwischen dem im Stichkanal 34 verbleibenden Kalibrierungsfluid und dem Blut B in der Kammer 33 bildet sich aufgrund der erfindungsgemäßen Eigenschaften dieses Fluids allenfalls eine Diffusionsspsannung < 1mV aus, die bei der Blutwertmessung keinen Meßfehler verursacht.

Ein dem Sensor 3 nach Figur 2 ähnlicher Sensor geht aus US 5 385 659 hervor. Jeder der in der Sensoreinrichtung 100 nach Figur 1 enthaltene Sensor 1, 2 usw. kann ein Sensor nach Art des Sensors 3 nach Figur 2 sein, soweit es potentiometrische Sensoren sind.

**Patentansprüche**

1.  Kalibrierungsfluid zum Kalibrieren eines Sensors (1, 2,3) zur Messung eines Blutwertes, das folgendes umfasst:

    einen biokompatiblen Elektrolyten, der bei 37°C folgendes aufweist:
    eine Konzentration von Hydrogencarbonationen von etwa 24 mmol/l $\pm$ 5 mmol/l
    einen pH-Wert im Bereich von 5 bis 9, inklusive einen Wert von 7,41,
    eine Ionenstärke von 155 mmol/l $\pm$ 10 mmol/l,

    und:
    wobei das genannte Fluid ferner einen organischen physiologischen Puffer umfasst, und weitere Ionentypen umfasst, die in normalem physiologischem Blut enthalten sind, wobei die Konzentrationen der Ionentypen in dem Fluid so wenig von die Konzentrationen dieser Ionentypen im Blut abweichen dass das Fluid bei einem Kontakt mit normalem physiologischen Blut eine Diffusionsspannung von weniger als 1 mV an einer Grenzfläche (35) zwischen dem Fluid und dem normalen physiologischen Blut hat.

2.  Fluid nach Anspruch 1, das eine Osmolarität im Bereich von 295 mosmol/l $\pm$ 20mosmol/l hat.

3.  Fluid nach Anspruch 1 oder 2, das einen pH-Wert im Bereich zwischen 6,6 und 8,0 hat.

4.  Fluid nach einem der vorhergehenden Ansprüche, bei dem der organische Puffer ein durch ein bestimmtesTris/TrisH$^+$-Verhältnis definierter Tris-Puffer ist.

5.  Fluid nach einem der Ansprüche 1 bis 3, bei dem der organische Puffer ein durch ein bestimmtes R-P0$_4{}^{2-}$R-HPO4$^-$-Verhältnis definierter organischen Phosphat Puffer ist, wobei R ein organisches Radikal ist.

6.  Fluid nach einem der vorhergehenden Ansprüche, **gekennzeichnet dadurch dass** die Summe aller Partialdrücke des Fluids kleiner als ein auf dem Fluid lastender Druck ist.

7.  Fluid nach Anspruch 6, **dadurch gekennzeichnet, dass** der Partialdruck von N$_2$ bei 37° C in dem Fluid geringer als der Wert dieses Drucks in normalem, physiologischem Blut bei dieser Temperatur ist.

8.  Fluid nach Anspruch 7, bei dem der N$_2$-Partialdruck des Fluids gleich null ist.

9.  Fluid nach Anspruch 1-4 oder 6-8, bestehend aus:

    130,00 mmol/l Na$^+$

5,00 mmol/l $K^+$
0,90 mmol/l $Mg^{2+}$
12,50 mmol/l Tris
12,50 mmol/l $TrisH^+$
113,20 mmol/l $Cl^-$
0,90 mmol/l $SO_4^{2-}$
9,82 mmol/l $Acetat^-$
0,24 mmol/l $CO_3^{2-}$
24,00 mmol/l $HCO_3^-$
pH-Wert = 7,80

$$pCO_2 = 14,1 \text{ mm Hg} = 14,1 \cdot (4/3) \cdot 10^2 \text{ Pa}$$

**10.** Fluid nach Anspruch 1-4 oder 6-3 bestehend aus:

129.80 mmol/l $Na^+$
5.00 mmol/l $K^+$
0.10 mmol/l $Ca^{2+}$
0.90 mmol/l $Mg^{2+}$
12.50 mmol/l Tris
12.50 mmol/l $TrisH^+$
113.20 mmol/l $Cl^-$
0.90 mmol/l $SO_4^{2-}$
9.82 mmol/l $Acetat^-$
0.24 mmol/l $CO_3^{2-}$
24.00 mmol/l $HCO_3^-$
pH-Wert = 7.80

$$pCO_2 = 14.1 \text{ mmHg} = 14,1 \cdot (4/3) \cdot 10^2 \text{ Pa}$$

**11.** Fluid nach Anspruch 1-3 oder 5-8 bestehend aus:

140,00 mmol/l $Na^+$
4,00 mmol/l $K^+$
1,20 mmol/l $Ca^{2+}$
0,60 mmol/l $Mg^{2+}$
8,35 mmol/l $R\text{-}PO_4^{2-}$
1,65 mmol/l $R\text{-}HPO_4^-$
102,99 mmol/l $Cl^-$
0,60 mmol/l $SO_4^{2-}$
1,00 mmol/l $Acetat^-$
0,03 mmol/l $CO_3^{2-}$
24,00 mmol/l $HCO_3^-$
pH-Wert = 6,9

$$pCO_2 = 112 \text{ mmHg} = 14,1 \cdot (4/3) \cdot 10^2 \text{ Pa}$$

**12.** Verwendung eines Fluids nach einem der vorhergehenden Ansprüche in einem eine Meßelektrode (31) zur Messung eines Blutwertes und eine Referenzelektrode (32) aufweisenden potentiometrischen Sensor (3) als Brücken-Elektrolyt zwischen der der Meßelektrode (31) und der Referenzelektrode (32).

13. Verfahren zur Herstellung eines Fluids nach einem der vorhergehenden Ansprüche, **dadurch gekennzeichnet, daß** in einer bestimmten Menge eines im wesentlichen salzfreien wässrigen Lösungsmittels NaHCO$_3$ zusammen mit zumindest einem anderen biokompatiblen Salz in einem solchen Gewichtsverhältnis zueinander gelöst werden, daß danach die Lösung bei 37°C eine Bikarbonat-Ionenkonzentration im normalen physiologischen Bereich des Blutes, einen pH-Wert in einem den Wert 7,41 enthaltenden pH-Wertebereich innerhalb von 2 bis 13 und eine Ionenstärke im normalen physiologischen Bereich des Blutes aufweist.

**Claims**

1. Calibration fluid for the calibration of a sensor (1, 2, 3) for measuring a blood value, comprising:

   a biocompatible electrolyte which at 37 °C exhibits:

   a concentration of hydrogencarbonate ions of 24 mmol/l $\pm$ 5 mmol/l,
   a pH value which is in the range of 5 to 9, including a value 7.41,
   and an ionic strength of 155 mmol/l $\pm$ 10 mmol/l, and

   wherein said fluid further comprises an organic physiological buffer and comprises further ion types which are contained in normal, physiological blood, the concentrations of said ion types in the fluid differing from the concentrations of these ion types in blood to such a low degree that upon contact with normal, physiological blood, the fluid has a diffusion voltage of less than 1 mV at an interface (35) between the fluid and the normal, physiological blood.

2. Fluid according to claim 1, which has an osmolarity in the range of 295 mosmol/l $\pm$ 20 mosmol/l.

3. Fluid according to either claim 1 or claim 2, which has a pH in the range between 6.6 and 8.0.

4. Fluid according to any one of the preceding claims, in which the organic buffer is a Tris buffer defined by a specific Tris/TrisH$^+$ ratio.

5. Fluid according to any one of claims 1 to 3, in which the organic buffer is an organic phosphate buffer defined by a specific R-PO$_4$$^{2-}$/R-HPO4$^-$ ratio, R being an organic radical.

6. Fluid according to any one of the preceding claims, **characterised in that** the sum of all partial pressures of the fluid is lower than a pressure which bears on the fluid.

7. Fluid according to claim 6, **characterised in that** the partial pressure of N$_2$ in the fluid at 37 °C is lower than the value of this pressure in normal, physiological blood at this temperature.

8. Fluid according to claim 7, in which the N$_2$ partial pressure of the fluid is equal to zero.

9. Fluid according to any one of claims 1-4 or 6-8, consisting of:

   | | |
   |---|---|
   | 130.00 mmol/l | Na$^+$ |
   | 5.00 mmol/l | K$^+$ |
   | 0.90 mmol/l | Mg$^{2+}$ |
   | 12.50 mmol/l | Tris |
   | 12.50 mmol/l | TrisH$^+$ |
   | 113.20 mmol/l | Cl$^-$ |
   | 0.90 mmol/l | SO$_4$$^{2-}$ |
   | 9.82 mmol/l | Acetate$^-$ |
   | 0.24 mmol/l | CO$_3$$^{2-}$ |
   | 24.00 mmol/l | HCO$_3$$^-$ |
   | pH = 7.80 | |

$$pCO_2 = 14.1 \text{ mm Hg} = 14.1 \cdot (4/3) \cdot 10^2 \text{ Pa}$$

**10.** Fluid according to any one of claims 1-4 or 6-8, consisting of:

| | |
|---|---|
| 129.80 mmol/l | $Na^+$ |
| 5.00 mmol/l | $K^+$ |
| 0.10 mmol/l | $Ca^{2+}$ |
| 0.90 mmol/l | $Mg^{2+}$ |
| 12.50 mmol/l | Tris |
| 12.50 mmol/l | $TrisH^+$ |
| 113.20 mmol/l | $Cl^-$ |
| 0.90 mmol/l | $SO_4^{2-}$ |
| 9.82 mmol/l | $Acetate^-$ |
| 0.24 mmol/l | $CO_3^{2-}$ |
| 24.00 mmol/l | $HCO_3^-$ |
| pH = 7.80 | |

$$pCO_2 = 14.1 \text{ mm Hg} = 14.1 \cdot (4/3) \cdot 10^2 \text{ Pa}$$

**11.** Fluid according to any one of claims 1-3 or 5-8, consisting of:

| | |
|---|---|
| 140.00 mmol/l | $Na^+$ |
| 4.00 mmol/l | $K^+$ |
| 1.20 mmol/l | $Ca^{2+}$ |
| 0.60 mmol/l | $Mg^+$ |
| 8.35 mmol/l | $R\text{-}PO_4^{2-}$ |
| 1.65 mmol/l | $R\text{-}HPO_4^-$ |
| 102.99 mmol/l | $Cl^-$ |
| 0.60 mmol/l | $SO_4^{2-}$ |
| 1.00 mmol/l | $Acetate^-$ |
| 0.03 mmol/l | $CO_3^{2-}$ |
| 24.00 mmol/l | $HCO_3^-$ |
| pH = 6.9 | |

$$pCO_2 = 112 \text{ mm Hg} = 14.1 \cdot (4/3) \cdot 10^2 \text{ Pa}$$

**12.** Use of a fluid according to any one of the preceding claims, in a potentiometric sensor (3) having a measuring electrode (31) for measuring a blood value and a reference electrode (32), as a bridging electrolyte between the measuring electrode (31) and the reference electrode (32).

**13.** Method for preparing a fluid according to any one of the preceding claims, **characterised in that** a specific quantity of a substantially salt-free, aqueous solvent $NaHCO_3$ is dissolved together with at least one other biocompatible salt, at a weight ratio relative to one another such that the solution subsequently exhibits, at 37 °C, a bicarbonate ion concentration in the normal physiological range of blood, a pH within a range of 2 to 13 including the value 7.41 and an ionic strength in the normal physiological range for blood.

**Revendications**

1. Fluide de calibrage destiné à étalonner un capteur (1, 2,3) pour la mesure d'une valeur sanguine, comprenant les éléments suivants:

   un électrolyte biocompatible qui donne à 37 °C les valeurs suivantes:

   une concentration en ions carbonates d'hydrogène de 24 mmol/l $\pm$ 5 mmol/l
   un indice de pH dans une plage de 5 à 9, incluant une valeur de 7,41,
   une force ionique de 155 mmol/l $\pm$ 10 mmol/l,

   et:

   où ledit fluide comprend en outre un tampon physiologique organique et comprend des types ioniques supplémentaires, qui se présentent dans la physiologie sanguine normale, où la concentration de ces types ioniques dans le fluide s'écarte si peu de la concentration de ces types ioniques dans le sang que le fluide entrant en contact avec du sang physiologique normal offre un potentiel de diffusion inférieur à 1 mV à l'interface (35) entre le fluide et le sang physiologique normal.

2. Fluide selon la revendication 1, qui présente une osmolarité avoisinant 295 mosmol/l $\pm$ 20 mosmol/l.

3. Fluide selon la revendication 1 ou la revendication 2, qui présente un indice de pH situé entre 6,6 et 8,0.

4. Fluide selon l'une des revendications précédentes, où le tampon organique est un tampon Tris défini par un certain rapport Tris/TrisH$^+$.

5. Fluide selon l'une des revendications 1 à 3, où le tampon organique est un tampon phosphate organique défini par un certain rapport R-PO$_4$$^{2-}$/R-HPO4$^-$, où R est un radical organique.

6. Fluide selon l'une des revendications précédentes, **caractérisé en ce que** la somme de toutes les pressions partielles dans le fluide est inférieure à une pression pesant sur le fluide.

7. Fluide selon la revendication 6, **caractérisé en ce que** la pression partielle de N$_2$ à 37 °C dans le fluide est inférieure à la valeur de cette pression dans le sang physiologique normal à la même température.

8. Fluide selon la revendication 7, où la pression partielle de N$_2$ dans le fluide est égale à zéro.

9. Fluide selon la revendication 1-4 ou 6-8, se composant de:

   130,00 mmol/l de Na$^+$
   5,00 mmol/l de K$^+$
   0,90 mmol/l de Mg$^{2+}$
   12,50 mmol/l de Tris
   12,50 mmol/l de TrisH$^+$
   113,20 mmol/l de Cl$^-$
   0,90 mmol/l de SO$_4$$^{2-}$
   9,82 mmol/l d'acétate$^-$
   0,24 mmol/l de CO$_3$$^{2-}$
   24,00 mmol/l de HCO$_3$$^-$
   indice de pH = 7,80

$$pCO_2 = 14{,}1 \text{ mm Hg} = 14{,}1.(4/3).10^2 \text{ Pa}$$

10. Fluide selon la revendication 1-4 ou 6-8, se composant de:

    129,80 mmol/l de Na$^+$

    5,00 mmol/l de $K^+$
    0,10 mmol/l de $Ca^{2+}$
    0,90 mmol/l de $Mg^{2+}$
    12,50 mmol/l de Tris
    12,50 mmol/l de $TrisH^+$
    113,20 mmol/l de $Cl^-$
    0,90 mmol/l de $SO_4^{2-}$
    9,82 mmol/l d'acétate$^-$
    0,24 mmol/l de $CO_3^{2-}$
    24,00 mmol/l de $HCO_3^-$
    indice de pH = 7,80

$$pCO_2 = 14{,}1 \text{ mm Hg} = 14{,}1.(4/3).10^2 \text{ Pa}$$

**11.** Fluide selon la revendication 1-3 ou 5-8, se composant de:

    140,00 mmol/l de $Na^+$
    4,00 mmol/l de $K^+$
    1,20 mmol/l de $Ca^{2+}$
    0,60 mmol/l de $Mg^{2+}$
    8,35 mmol/l de $R\text{-}PO_4^{2-}$
    1,65 mmol/l de $R\text{-}HPO_4^-$
    102,99 mmol/l de $Cl^-$
    0,60 mmol/l de $SO_4^{2-}$
    1,00 mmol/l d'acétate$^-$
    0,03 mmol/l de $CO_3^{2-}$
    24,00 mmol/l de $HCO_3^-$
    indice de pH = 6,9

$$pCO_2 = 112 \text{ mm Hg} = 14{,}1.(4/3).10^2 \text{ Pa}$$

**12.** Utilisation d'un fluide selon l'une des revendications précédentes dans un capteur potentiométrique (3), comportant une électrode de mesure (31) destinée à mesurer une valeur sanguine et une électrode de référence (32), en tant qu'électrolyte intermédiaire entre l'électrode de mesure (31) et l'électrode de référence (32).

**13.** Procédé de production d'un fluide selon l'une des revendications précédentes, **caractérisé en ce que** une certaine quantité de solvant aqueux $NaHCO_3$ essentiellement exempt de sel est mélangée en solution à au moins un autre sel biocompatible selon un tel rapport pondéral que la solution puisse ensuite présenter à 37 °C une concentration en ions bicarbonates correspondant à la plage physiologique normale du sang, un indice de pH dans une fourchette de pH allant de 2 à 13 incluant la valeur de 7,41 et une force ionique située dans la plage physiologique normale du sang.

# FIG 1

BF — 15

18

C

100

1

2

12

16

19

111

B

10

11

BF, C, C'

110

# FIG 2

30  3

BF, C, C'

BF, C, C', B  33

32  34  35  31

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0657030 B1
- EP 0362032 A2
- FR 2436991 A
- EP 0571066 A
- EP 0657030 A
- EP 0790499 A2
- US 5385659 A

**In der Beschreibung aufgeführte Nicht-Patentliteratur**

- **KORYTA.** Medical and Biological Applications of Electrochemical Devices. John Wiley & Sons, 1980, 82
- **MÜLLER-PLATHE.** Säure-Basen-Haushalt und Blutgase. Thieme Verlag, 1982, 32